# DEMANDE DE BREVET EUROPEEN

(11) **EP 0 781 531 A1**
(43) Date de publication de la demande: **02.07.1997**
(21) Numéro de dépôt: 96402906.0
(22) Date de dépôt: 26.12.1996
(51) Int. Cl.: A61F 2/28, A61C 8/00

(54) **Procédé et dispositif pour faciliter la croissance osseuse, notamment en chirurgie dentaire et en chirurgie maxillo-faciale et dispositif pour la mise en oeuvre de ce procédé**

(30) Priorité: 28.12.1995 FR 9515675
(71) Demandeur: Derycke, Raymond-René, 75116 Paris (FR)
(72) Inventeur: Derycke, Raymond-René, 75116 Paris (FR)
(74) Mandataire: Bernasconi, Jean

(57) **Abrégé**

Procédé et dispositif pour faciliter la croissance osseuse, notamment en chirurgie dentaire et en chirurgie maxillo-faciale et dispositif pour la mise en oeuvre de ce procédé.

Le dispositif est caractérisé en ce qu'il comporte un moyen élastique (1), interposé entre l'os et une membrane disposée sous les tissus sus-jacents, et susceptible de soulever la membrane d'une faible distance avec une pression de valeur contrôlée, des moyens étant prévus pour maintenir temporairement, jusqu'à la cicatrisation, ledit moyen élastique (1) dans un état dans lequel il présente une surépaisseur minime entre la surface osseuse et la membrane et n'exerce sur celle-ci aucun effort sensible.

## Description

La présente invention a trait à un procédé pour favoriser la croissance osseuse, notamment en chirurgie dentaire ou en chirurgie maxillo-faciale, ainsi qu'à un dispositif permettant la mise en oeuvre de ce procédé.

Il est souvent nécessaire, ou désirable, après un événement traumatique ou dégénératif, ou encore en vue de la pose d'une prothèse ou d'un implant, de provoquer une croissance osseuse à la surface d'un os tel que, par exemple, une mandibule, un maxillaire ou un os du massif facial.

La technique chirurgicale utilisée à cette fin consiste à écarter de la surface osseuse à partir de laquelle doit s'effectuer la croissance osseuse, et donc le gain en épaisseur, un lambeau périosté afin de créer, entre la surface osseuse et le tissu sus-jacent, un espace dans lequel pourra s'effectuer la croissance osseuse.

A cette fin, après avoir éliminé ou séparé le périoste d'avec la surface osseuse sous-jacente, puis soulevé l'ensemble du tissu sus-jacent à l'os, on interpose généralement entre la surface osseuse dégagée et les lambeaux ou tissu sus-jacents, une membrane non perméable, sous laquelle se fera la croissance osseuse. Pour créer l'espace nécessaire à la croissance osseuse on cherche à écarter cette membrane de la surface osseuse sous-jacente, ce qui peut cependant présenter des risques d'infection ou de nécrose fibreuse et rend plus difficile ou aléatoire une bonne fermeture et une bonne cicatrisation de la plaie, après la mise en place de la membrane.

Généralement l'obtention du volume sous-jacent à la membrane pour permettre la croissance osseuse est assuré par un comblement osseux, ce qui nécessite mormalement un prélèvement sur le patient, ce qui complique et prolonge l'intervention.

Dans les conditions habituelles, compte tenu de toutes ces difficultés, un gain osseux de l'ordre de 2 à 3 mm est considéré comme un bon résultat et un gain de l'ordre de 5 mm comme un résultat exceptionnel.

La présente invention se propose de remédier à ces inconvénients et de fournir un procédé qui permette d'améliorer la croissance osseuse, d'augmenter le gain en épaisseur, de supprimer la nécessité d'un comblement osseux, et de réduire les risques de mauvaise fermeture ou cicatrisation et les risques de fibrose.

L'invention a donc pour objet un procédé pour faciliter la croissance osseuse, notamment en chirurgie dentaire ou en chirurgie maxillo-faciale, dans lequel, après avoir dépériosté l'os à partir duquel la croissance en épaisseur doit s'effectuer, on interpose, entre l'os et les tissus sus-jacents, une membrane destinée à délimiter un volume à la surface de l'os, dans lequel peut se faire la croissance osseuse, caractérisé en ce que, après avoir vérifié la bonne fermeture et cicatrisation des tissus sus-jacents, on applique, sur la membrane, un effort ou pression d'intensité contrôlée pour créer, entre l'os et la membrane, un volume suffisant pour une bonne croissance osseuse.

Il est ainsi possible, après l'intervention, de laisser les tissus sus-jacents se cicatriser et d'obtenir une bonne qualité de ce tissus, limitant les risques de nécrose, puis, une fois ce résultat obtenu, de réaliser, entre le tissu et l'os dépériosté, un volume permettant la croissance osseuse.

L'invention a également pour objet un dispositif implantable pour la mise en oeuvre de ce procédé caractérisé en ce qu'il comporte un moyen élastique, interposé entre l'os et la membrane, et susceptible de soulever la membrane d'une faible distance avec une pression de valeur contrôlée, des moyens étant prévus pour maintenir temporairement, jusqu'à la cicatrisation, ledit moyen élastique dans un état dans lequel il présente une surépaisseur minime entre la surface osseuse et la membrane et n'exerce sur celle-ci aucun effort sensible.

Dans une forme de réalisation particulièrement préférée, ledit moyen comporte un ressort de forme plate et de très faible épaisseur, réalisé,de préférence, en un matériau à mémoire de forme et agencé pour être interposé, en créant une faible surépaisseur, entre l'os et le tissu sus-jacent alors qu'il est dans un état rétracté, c'est-à-dire sous contrainte, des moyens étant prévus pour permettre audit ressort, au moment souhaité, de se déformer sous l'action de son élasticité et de soulever la membrane en tendant le tissu sus-jacent, et ceci avec un effort contrôlé.

Le matériau à mémoire de forme peut être avantageusement un matériau ou alliage métallique et l'on préfère particulièrement les alliages nickel-titane en proportions égales.

Le matériau peut ainsi être avantageusement choisi pour rester dans sa position neutre rétractée à une température inférieure à la température naturelle du corps humain, par exemple à une température de l'ordre de 20°C, et, lorsqu'il est porté à une température supérieure, par exemple de l'ordre de 35°C, reprendre ses propriétés élastiques et chercher à se détendre en prenant appui sur la surface osseuse et en exerçant une tension sur le tissus sus-jacent.

Cependant, ledit moyen élastique peut aussi être constitué de, ou incorporer, un ressort, par exemple en métal, qui ne possède pas de mémoire de forme.

De préférence le ressort est agencé pour exercer sur la membrane une pression qui ne dépasse pas 1333 à 4665 Pa (10 à 35 mm Hg), et s'écarter de l'os sur lequel il prend appui d'une distance de l'ordre de 5 à 15 mm.

De façon avantageuse l'épaisseur du ressort, sous contrainte, n'est pas supérieure à 1 à 3 mm.

Afin de maintenir le ressort dans sa position aplatie, une fois qu'il a été mis en place et qu'il se trouve réchauffé à la température du corps humain, on prévoit avantageusement un moyen de blocage, tel que, par exemple, une suture libérable depuis l'extérieure grâce à un fil de suture sortant de la plaie et susceptible d'être actionné par le chirurgien depuis l'extérieur. Il suffit alors de tirer sur la suture pour la dégager et libérer le ressort.

Le ressort peut avoir toute forme convenable, par exemple une forme de zigzag normalement maintenue dans un plan à l'état refroidi, ou bien encore une forme en spirale, mais aussi, bien entendu, toutes les formes permettant le soulèvement de la membrane une fois que les moyens de maintien temporaire ont été libérés, et, s'il y a lieu, que la température de libération a été atteinte et le ressort effectivement libéré, avec la pression voulue.

De préférence le ressort peut être constitué d'un fil replié en zigzag et contenu, à l'état rétracté, dans un plan. Lorsque la température s'approche de la température normale du corps de 37°, supérieure à la température de transition, le fil se déforme en prenant appui sur ses extrémités et soulève la membrane sus-jacente, en lui donnant une forme de coupole plus ou moins sphérique, ou bien, dans une autre forme de réalisation, de portion de cylindre.

Dans une autre forme de réalisation le fil-ressort peut avoir une forme de spirale, par exemple carrée ou circulaire.

Le moyen élastique peut aussi être constitué d'une nappe élastique, continue ou ajourée, ou d'un treillis, ou d'une pluralité de ressorts associés, ce moyen étant tel qu'il tende à se soulever et s'incurver, au moins dans sa partie centrale, lorque le moyen qui le maintient temporairement à plat, est relaché.

Le dispositif selon l'invention peut également être réalisé sous forme de soufflet ou accordéon élastique mobile entre une position comprimée et une position détendue. Ce soufflet peut être formé, par exemple, par déformation de la partie centrale d'une feuille métallique de faible épaisseur, la périphérie non déformée de la feuille pouvant être modelée et découpée par le chirurgien pour l'adapter au tissus sur lequel le dispositif prend appui. Dans de telles formes de réalisation, le soufflet élastique fait également office de membrane, de sorte qu'une membrane associée n'est plus nécessaire.

De préférence l'encombrement en longueur (X) et en largeur (Y) du dispositif selon l'invention est de l'ordre de 3 à 40 mm.

L'encombrement en hauteur (Z) est de préférence de l'ordre de 1 à 4 mm et très avantageusement ne dépasse pas 2 mm.

La force exercée par le ressort, compte tenu des surfaces osseuses habituellement concernées en chirurgie dentaire ou faciale, peut aller, par exemple, de 5 à 200 g, et de préférence de 30 à 100 g.

Il est important d'éviter que le moyen élastique ou ressort ne se déplace en glissant le long de la surface osseuse et ne s'éloigne de la zone prévue pour la croissance. A cette fin il est préférable de fixer le ressort. On peut, par exemple, fixer le ressort contre l'os sous-jacent par une ou plusieurs parties du ressort qui restent dans le plan initial après déformation. Cette fixation peut s'effectuer, par exemple par des agrafes, vis ou tout autre moyen usuel. On peut également, en variante, fixer le ressort en le suturant à un élément de tissus sus-jacent pour éviter qu'il ne puisse se déplacer en translation parallèlement à la surface osseuse.

De façon avantageuse, les extrémités du ressort, qui sont formées pour s'appuyer sur le tissu dur sous-jacent, présentent des boucles, oeillets, ou autres formes permettant leur ancrage, par exemple par des vis ou agrafes à fixer dans le tissu osseux.

Le ressort peut également être fixé à l'avance à la membrane et être ainsi maintenu en place par la membrane elle-même, celle-ci étant, ou non, suturée au tissus sus-jacent. Cette fixation à la membrane peut être réalisée à l'avance ou bien être décidée sur place par le chirurgien.

Par exemple, le ressort, s'il est fait en fil, peut être incorporé dans une fine membrane, par exemple en silicone.

D'autres avantages et caractéristiques de l'invention apparaîtront à la lecture de la description suivante, faite à titre d'exemple non limitatif et se référant au dessin annexé dans lequel :
- la figure 1 représente une vue de dessus d'un ressort selon l'invention ;
- la figure 2 représente une vue de bout de ce ressort ;
- la figure 3 représente une vue de profil schématique de ce ressort à l'état maintenu ;
- la figure 4 représente une vue de profil schématique de ce ressort à l'état libéré ;
- la figure 5 est une vue en perspective d'un autre exemple de réalisation du dispositif selon l'invention ;
- la figure 6 représente une vue de face du dispositif de la figure 5 à l'état déplié, et
- la figure 7 est une vue en perspective d'une autre variante du dispositif selon l'invention.

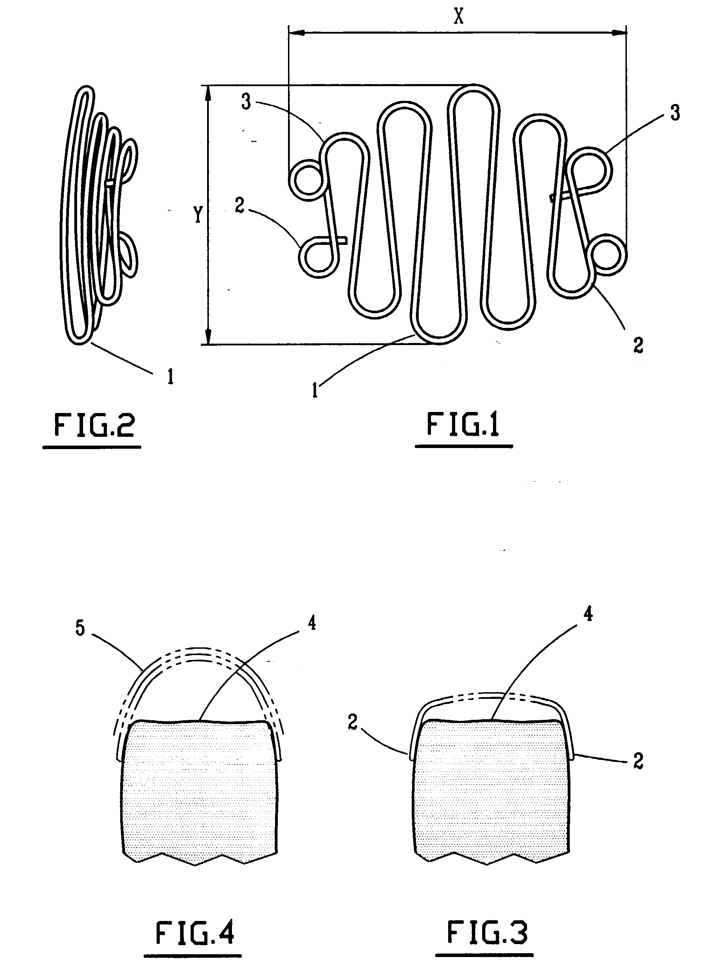

On a représenté sur le dessin un exemple de dispositif selon l'invention dans lequel le moyen élastique est un ressort réalisé en un fil d'alliage nickel-titane ayant un diamètre de préférence égal à une valeur comprise entre 0,43 et 0,83 mm. Comme on le voit bien sur la figure 1, ce ressort possède, lorsqu'on le voit de dessus, une forme en zigzag avec des longueurs de brins maximales dans la partie centrale et diminuant progressivement vers les deux extrémités. Les deux extrémités du fil 1 sont courbées pour former deux paires de boucles 2, 3. On a désigné par X et Y les encombrements en longueur et en largeur de ce ressort.

On voit ce ressort à l'état maintenu sur la figure 3. Il présente un profil relativement plat, les extrémités au niveau des boucles 2, 3 étant par contre davantage incurvées ce qui permet de le fixer, par exemple par des agrafes, contre les deux pans osseux qui bordent la surface osseuse 4 que l'on veut surélever, par exemple un os maxillaire. Par ailleurs, comme on le voit sur la figure 2, les différents bras sont également incurvés dans un plan perpendiculaire à la longueur X du ressort, c'est-à-dire dans la direction Y, et l'on préfère que la différence de niveau, dans la direction de la hauteur Z du ressort pour un même brin du ressort en zigzag ne dépasse pas 2 mm environ.

Dans l'exemple représenté les dimensions maximales X et Y sont respectivement de 20 et 16 mm lorsque le ressort est rétracté par un moyen le maintenant sensiblement à plat ou bien lorsqu'il est refroidi à une température inférieure à la température de libération déterminée par le choix de l'alliage, par exemple 20°C. Au contraire lorsqu'il est libéré le même ressort présente des dimensions X de 17,5 mm et Y de 15 mm.

A froid, ou à l'état rétracté, c'est-à-dire dans l'état représenté sur la figure 3, l'encombrement du ressort au-dessus de la surface osseuse 4 est de l'ordre de 2 mm. Lorsque le ressort est libéré, comme on le voit sur la figure 4, la hauteur du ressort au-dessus de l'os est de 7 mm et il soulève ainsi une membrane 5 sus-jacente qui a été interposée entre le ressort, lorsqu'il était à plat, et les tissus sus-jacents.

Le ressort, fabriqué dans cet alliage à mémoire de forme présente, à froid, par exemple à la température de stockage de +4°C, l'allure aplatie représentée sur la figure 3. Le chirurgien, après avoir dégagé et dépériosté la surface osseuse 4, se saisit du ressort froid et le fixe rapidement par les boucles 2, 3 de ses deux extrémités, contre les parois osseuses de part et d'autre de la surface osseuse 4. Afin de maintenir le ressort dans sa position aplatie, il passe immédiatement des fils de suture sur le ressort, par exemple dans la direction X, ce ou ces fils de suture étant bloqués par un noeud chirurgical qui peut être défait par traction sur le brin de fil qui dépassera de la plaie. De cette façon bien que le ressort parvienne rapidement à la température de 37,5°, il conserve sensiblement sa forme aplatie. Le chirurgien dispose sur le ressort une membrane de type classique destinée à la création du vide pour la croissance osseuse puis il ramène les lambeaux de tissu et referme les plans sus-jacents.

Une fois la plaie convenablement cicatrisée, en général quelques semaines après la pose, le chirurgien tire sur la partie dépassante de la suture et libère le noeud chirurgical. Le ressort se détend immédiatement et adopte alors la position incurvée représentée sur la figure 4 où il soulève la membrane 5, créant entre la membrane et la surface osseuse un espace vide d'une hauteur de l'ordre de 5 à 7 mm.

Bien entendu le dispositif selon l'invention pourrait aussi comprendre des moyens de maintien temporaires propres, tels que par exemple des fils rejoignant les boucles 2 et 3 les unes aux autres sur la longueur X du ressort et qui pourraient présenter un brin que le chirurgien laissera sortir de la plaie pour libérer le ressort. En variante de tels moyens de maintien, fils ou autres, peuvent être prévus à l'avance et seront libérés par le chirurgien après que celui-ci aura posé le ou les fils de suture qui assureront la poursuite du maintien à l'état aplati du ressort selon l'invention.

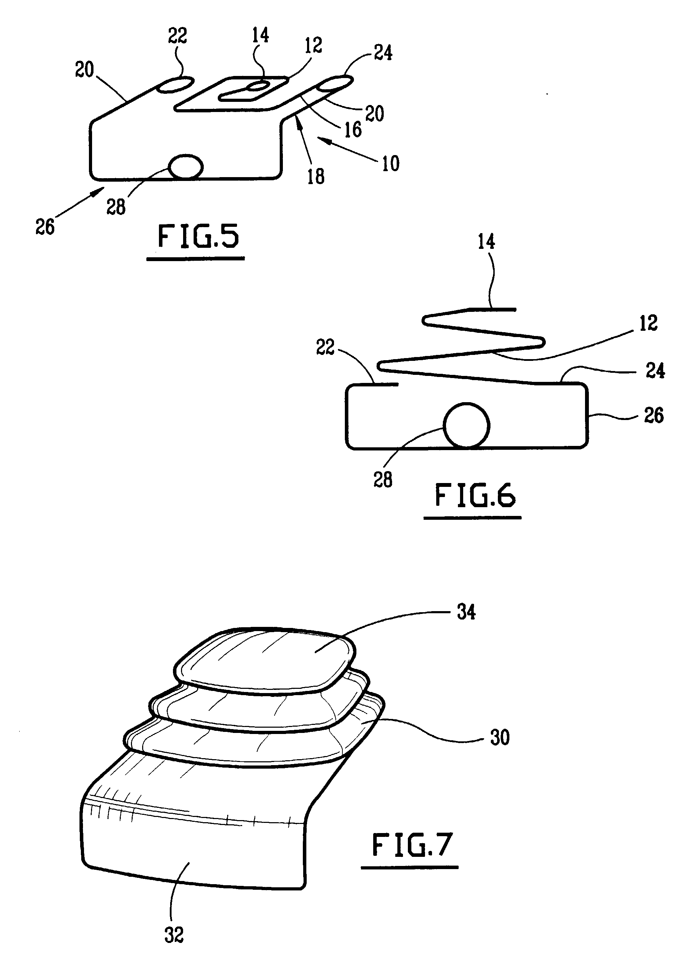

La figure 5 montre un autre exemple de réalisation du dispositif selon l'invention. Cet exemple se distingue essentiellement par le fait que le moyen élastique est un fil-ressort 10 dont la partie formant ressort 12 possède une forme d'une spirale carrée. En son centre, le ressort 12 se termine en une boucle 14 centrale de fixation destinée à maintenir le ressort à plat lors de la première phase de l'intervention chirurgicale.

A sa périphérie, le ressort 12 se termine en une spire périphérique 16 qui se prolonge en une partie 18 faisant office de base de maintien du ressort 12 contre la surface osseuse. A cet effet, la base de maintien 18 comporte d'une part une partie 20 destinée à être fixée sur la surface osseuse au moyen de deux boucles de fixation 22 et 24 disposées de part et d'autre du ressort 12, et d'autre part une partie 26, rabattue à 90° et destinée à être fixée contre un pan osseux au moyen d'une boucle centrale 28.

Pour la réalisation de ce dispositif, on utilise le même métal que celui ayant servi à la fabrication du dispositif des figures 1 et 2. Il est réalisé par la mise en forme d'un seul fil métallique dans lequel on forme d'abord la boucle 14, on enroule ensuite le fil pour obtenir la spirale formant le ressort 12, puis on réalise à l'extrémité de la spire périphérique 16 la boucle de fixation 24. Par la suite, on forme le fil de sorte qu'il s'étende parallèlement à la surface osseuse, on réalise deux pliages à 90°, on forme la boucle 28 au niveau du ressort 12 et on réalise encore deux pliages à 90° pour former la partie 26. Après le dernier pliage, le fil métallique est à nouveau parallèle à la surface osseuse et on réalise la dernière boucle 22 de fixation.

Les différentes phases de l'intervention chirurgicale sont analogues à celles décrites en regard des figures 1 à 4. Une fois la plaie convenablement cicatrisée, le chirurgien libère le ressort 12 qui prend une forme générale d'un tronc de pyramide pour soulever une membrane non-représentée. Sur la figure 6, le dispositif est représenté en vue de face avec la partie de ressort 12 à l'état déplié.

Bien entendu, le ressort peut également posséder une forme d'une spirale circulaire.

Avantageusement, on prévoit d'incorporer le dispositif 10 dans une fine membrane, par exemple en silicone, de sorte qu'une membrane associée supplémentaire n'est plus nécessaire.

La figure 7 montre encore une autre variante du dispositif selon l'invention. Selon cette variante, le dispositif est réalisé sous la forme d'un soufflet 30 élastique réalisé entièrement en métal. Avantageusement, on utilise le même métal que celui utilisé pour la réalisation des autres variantes. Le soufflet 30 est déplaçable entre une position comprimée, aplatie pour la première phase de l'intervention chirurgicale et une position détendue pour la deuxième phase afin de pouvoir soulever le tissus sus-jacent.

A sa base, le soufflet 30 est équipé d'une jupe 32 destinée à être découpée et modelée par le chirurgien pour l'adapter au tissu sur lequel le dispositif prend appui.

Par ailleurs, le soufflet 30 se termine à son sommet par une surface légèrement bombée 34.

Avantageusement, le soufflet 30 fait également office de membrane de sorte qu'une membrane associée supplémentaire n'est plus nécessaire.

D'une manière analogue aux autres exemples de réalisation, le soufflet 30 est maintenu comprimé lors d'une première phase chirurgicale par des moyens de maintien non représentés et est libéré après une période de cicatrisation afin de soulever les tissus sus-jacents.

## Revendications

1. Dispositif implantable pour faciliter la croissance osseuse, notamment en chirurgie dentaire et en chirurgie maxillo-faciale, caractérisé en ce qu'il comporte un moyen élastique (1), interposé entre l'os (4) et une membrane (5) disposée sous les tissus sus-jacents, et susceptible de soulever la membrane d'une faible distance avec une pression de valeur contrôlée, des moyens étant prévus pour maintenir temporairement, jusqu'à la cicatrisation, ledit moyen élastique (1) dans un état dans lequel il présente une surépaisseur minime entre la surface osseuse et la membrane et n'exerce sur celle-ci aucun effort sensible.

2. Dispositif selon la revendication 1, caractérisé en ce que ledit moyen élastique est réalisé en un matériau à mémoire de forme.

3. Dispositif selon l'une des revendications 1 et 2, caractérisé en ce que ledit moyen élastique comporte un ressort (1) de forme plate et de très faible épaisseur, agencé pour être interposé, en créant une faible surépaisseur, entre l'os et le tissu sus-jacent alors qu'il est dans un état rétracté, des moyens étant prévus pour permettre audit ressort (1), au moment souhaité, de se déformer sous l'action de son élasticité et de soulever la membrane (5) en tendant le tissu sus-jacent, et ceci avec un effort contrôlé.

4. Dispositif selon l'une des revendications 2 et 3, caractérisé en ce que le matériau à mémoire de forme est avantageusement un matériau ou alliage métallique notamment un alliage nickel-titane en proportions égales.

5. Dispositif selon la revendication 4, caractérisé par un matériau qui peut rester dans sa position neutre rétractée à une température inférieure à la température naturelle du corps humain, notamment à une température de l'ordre de 20°C.

6. Dispositif selon l'une des revendications 1 à 5, caractérisé en ce que ledit moyen élastique est agencé pour exercer sur la membrane une pression qui ne dépasse pas 1333 à 4665 Pa (10 à 35 mm Hg), et s'écarter de l'os sur lequel il prend appui d'une distance de l'ordre de 5 à 15 mm.

7. Dispositif selon l'une des revendications 1 à 6, caractérisé en ce que l'encombrement en longueur et en largeur du dispositif selon l'invention est de l'ordre de 3 à 40 mm.

8. Dispositif selon l'une des revendications 1 à 7, caractérisé en ce que l'épaisseur du moyen élastique, sous contrainte, n'est pas supérieure à 1 à 3 mm.

9. Dispositif selon l'une des revendications 1 à 8, caractérisé en ce que lesdits moyens de maintien comportent une suture libérable depuis l'extérieure grâce à un fil de suture sortant de la plaie et susceptible d'être actionné par le chirurgien depuis l'extérieur.

10. Dispositif selon l'une des revendications 3 à 9, caractérisé par un ressort en fil replié en forme de zigzag normalement maintenu sensiblement dans un plan.

11. Dispositif selon l'une des revendications 1 à 10, caractérisé en ce qu'il comporte, à deux extrémités, des moyens de fixation (2, 3) sur le tissu sous-jacent.

12. Dispositif selon la revendication 11, caractérisé en ce que lesdits moyens de fixation sont des boucles de fil (2,3).

13. Dispositif selon l'une quelconque des revendications 3 à 9, caractérisé par un ressort en fil replié en forme d'une spirale carrée ou circulaire normalement maintenu sensiblement dans un plan.

14. Dispositif selon l'une des revendications 1 et 2, caractérisé en ce que ledit moyen élastique comporte un soufflet élastique, mobile entre une position comprimée et une position détendue pour être interposé, en créant une faible surépaisseur, entre l'os et le tissus sus-jacent alors qu'il est dans la position comprimée, des moyens étant prévus pour permettre audit soufflet (30), au moment souhaité, de se déformer sous l'action de son élasticité vers la position détendue et de soulever et tendre le tissu sus-jacent, et ceci avec un effort contrôlé.
